# EUROPEAN PATENT APPLICATION

(11) **EP 4 830 764 A1**
(43) Date of publication of application: **28.10.2026**
(21) Application number: 25383096.2
(22) Date of filing: 14.10.2025
(51) Int. Cl.: A61N 1/06, A61H 19/00, A61H 21/00, A61N 1/32, A61N 1/40, A61N 1/36

(54) **THERAPEUTIC INTRACAVITARY DEVICE**

(30) Priority: 31.10.2024 ES 202430893
(71) Applicant: Capenergy Medical, S.L., 08970 Sant Joan Despi (ES)
(72) Inventor: SANCHEZ JAIME, MARIA DEL PILAR, 08970 SANT JOAN DESPI (ES)
(74) Representative: Isern Patentes y Marcas S.L.

(57) **Abstract**

The present invention relates to a therapeutic intracavitary device, enabled for use in a human or animal anatomical cavity; comprising generation means for generating electromagnetic radiation, application means for applying said electromagnetic radiation, and vibration means; the generation means and the application means being mutually enabled for transmission of the electromagnetic radiation to the application means; said application means being enabled for communication and irradiation of the electromagnetic radiation in the anatomical cavity; wherein the vibration means or vibrator are enabled for generation of a vibration and for communication of the vibration in the same anatomical cavity.

## Description

### OBJECT OF THE INVENTION

The object of the present application for invention is to register a therapeutic intracavitary device, which incorporates significant innovations and advantages over the techniques used to date.

More specifically, the invention proposes the development of a therapeutic intracavitary device, which, due to its particular arrangement, allows therapeutic treatments to be carried out in different human or animal anatomical cavities, while simultaneously dosing electromagnetic radiation and applying vibration in the same anatomical cavity.

### BACKGROUND OF THE INVENTION

A great multiplicity of different pathologies in different human or animal anatomical cavities, such as vagina, rectum, mouth, armpits, etc. are well known in the current state of the art.

For this purpose, different treatments in human or animal anatomical cavities, in other words, treatments that carried out inside such body cavities, are therefore also known. Such treatments are very susceptible to major improvements, especially for therapies that are carried out inside a body cavity, such as the vagina or rectum, for example.

Another object of the present invention is an active intracavitary accessory for applying diathermy by radio frequency, applied by contact electrodes in the body cavities, such as in the mouth, rectum or vagina.

The present invention contributes to solving and overcoming this problem, as it allows therapeutic treatments to be carried out in different human or animal anatomical cavities, while simultaneously dosing electromagnetic radiation and applying vibration in the same anatomical cavity. This invention is applicable in different medical specialities and especially, but not limited to: urology, gynaecology, physiotherapy and dentistry.

### DESCRIPTION OF THE INVENTION

The present invention has been developed in order to provide a therapeutic intracavitary device, enabled for use in a human or animal anatomical cavity; comprising generation means for generating electromagnetic radiation, application means for applying said electromagnetic radiation, and vibration means; the generation means and the application means being mutually enabled for transmission of the electromagnetic radiation to the application means; said application means being enabled for communication and irradiation of the electromagnetic radiation in the anatomical cavity; wherein the vibration means or vibrator are enabled for generation of a vibration and for communication of the vibration in the same anatomical cavity.

Preferably, in the therapeutic intracavitary device, the application means comprise at least one electrode element.

Additionally, in the therapeutic intracavitary device, the generation means, the application means and the vibrator are linked in data communication with control means that can control them.

Preferably, the therapeutic intracavitary device comprises manual gripping means, said gripping means and the application means and/or the vibrator being mutually enabled for manual operation.

Preferably, in the therapeutic intracavitary device, the gripping means, the application means and the vibrator are arranged and positioned in a single body.

Preferably, in the therapeutic intracavitary device, said body further incorporates the generation means.

Preferably, in the therapeutic intracavitary device, the gripping means comprise a knob or handle included or integrated in the body.

Additionally, in the therapeutic intracavitary device, the control means are arranged on the body.

Alternatively, in the therapeutic intracavitary device, such body incorporates a battery power supply.

Additionally, in the therapeutic intracavitary device, the control means are programmable and incorporate a program or software enabled for control over the generation means, the application means and the vibrator.

Preferably, in the therapeutic intracavitary device, the control means incorporate user interface means.

Alternatively, in the therapeutic intracavitary device, the application means and the vibrator are physically linked and therefore mutually integrated.

Alternatively, in the therapeutic intracavitary device, the generation means and the application means are mutually linked for transmission of the electromagnetic radiation from the generation means to the application means by a cable.

Alternatively, in the therapeutic intracavitary device, the generation means and the application means are mutually linked for transmission of the electromagnetic radiation from the generation means to the application means by wireless means.

Alternatively, in the therapeutic intracavitary device, the electrodes may be applied to the skin.

Alternatively, in the therapeutic intracavitary device, the electrodes may be insulated or non-insulated.

Alternatively, in the therapeutic intracavitary device, the electrodes may be of the active and/or passive type.

Alternatively, in the therapeutic intracavitary device, the electrodes are monopolar or bipolar return electrodes.

Alternatively, in the therapeutic intracavitary device, the control means are linked to the generation means, the application means and the vibrator by a cable.

Alternatively, in the therapeutic intracavitary device, the control means are linked to the generation means, the application means and the vibrator by wireless means.

Alternatively, in the therapeutic intracavitary device, the control means are enabled for control from a mobile phone or similar.

Alternatively, in the therapeutic intracavitary device, the control means are enabled to include a removable storage memory card for storing particular details of treatments of different patients.

Alternatively, in the therapeutic intracavitary device, the generation means, the application means and the vibrator are enabled and installed for automatic and/or remote and/or robotic operation controlled from the control means.

Alternatively, in the therapeutic intracavitary device, the interface means are linked to the control means by a wireless connection.

Alternatively, the therapeutic intracavitary device incorporates a temperature sensor.

Alternatively, in the therapeutic intracavitary device, the temperature sensor is linked in data communication with the control means.

Alternatively, in the therapeutic intracavitary device, the temperature sensor is linked in data communication with the application means and/or with the vibrator.

Alternatively, in the therapeutic intracavitary device, the generation means and the control means are mutually integrated.

Alternatively, in the therapeutic intracavitary device, the electromagnetic radiation is by radio frequency.

Alternatively, in the therapeutic intracavitary device, the electromagnetic radiation applied from the application means is at a frequency between 400 KHz and 3 MHz.

The present invention further relates to a method of therapeutic intracavitary treatment, designed for therapeutic treatment of a human or animal anatomical cavity, comprising irradiation of electromagnetic radiation in the anatomical cavity and at the same time applying vibration on the same anatomical cavity.

Alternatively, in the method of therapeutic intracavitary treatment, the electromagnetic radiation is by radio frequency.

Alternatively, in the method of therapeutic intracavitary treatment, the irradiated electromagnetic radiation is at a frequency between 400 KHz and 3 MHz.

Due to the present invention, therapeutic treatments can be carried out in different human or animal anatomical cavities, while simultaneously dosing electromagnetic radiation and applying vibration in the same anatomical cavity.

The present invention proposes an intracavitary use, which is particularly applicable for use with molecular bio-resonance therapy equipment, which is especially developed for intracavitary treatments in gynaecology and urology and which characteristically has a number of innovative features in its structural configuration and electrode arrangement, which optimise the treatment.

Furthermore, it can incorporate various models of electrodes of different sizes, especially at the end part, to adapt to different types of treatment.

The applicant is not aware of a device which is simultaneously enabled for therapeutic treatment based on electromagnetic irradiation or radio frequency irradiation and at the same time applying vibration.

The therapeutic intracavitary device of the proposed invention, both in its design and structural configuration and in the constitution and arrangement of the elements it incorporates, notably improves the performance, versatility and effectiveness of the same with respect to similar accessory elements currently existing in the state of the art for the same purpose.

Other characteristics and advantages of the therapeutic intracavitary device will be apparent from the description of a preferred, but not exclusive, embodiment, which is illustrated by way of non-limiting example in the accompanying drawings, in which:

### BRIEF DESCRIPTION OF THE DRAWINGS

Figure 1 is a schematic and conceptual view of a preferred embodiment of the therapeutic intracavitary device of the present invention.
Figure 2 is another schematic and conceptual view of another preferred embodiment of the therapeutic intracavitary device of the present invention.
Figure 3 is another schematic view of another preferred embodiment of the therapeutic intracavitary device of the present invention.
Figure 4 is another schematic and conceptual view of another preferred embodiment of the therapeutic intracavitary device of the present invention.
Figure 5 is another schematic and conceptual view of another preferred embodiment of the therapeutic intracavitary device of the present invention.

### DESCRIPTION OF A PREFERRED EMBODIMENT

The therapeutic intracavitary device of the proposed invention is designed and enabled for use in a human or animal anatomical or body cavity, and has the particularity and advantage that it is enabled for therapeutic treatment based simultaneously on electromagnetic irradiation in a body cavity and on application of a vibration in the same body cavity.

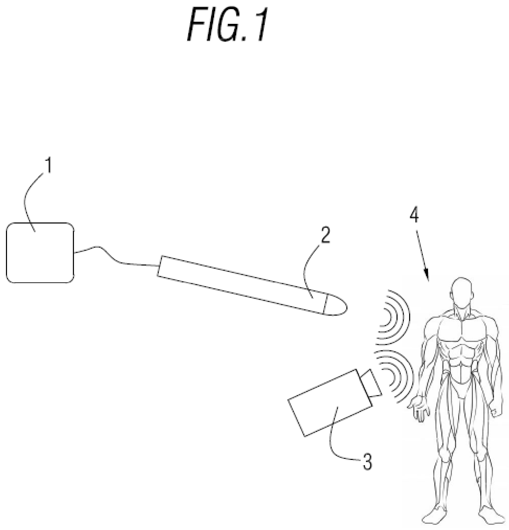

Accordingly, and as schematically shown in figure 1, the therapeutic intracavitary device of the invention comprises generation means 1 for generating electromagnetic radiation, application means 2 for applying said electromagnetic radiation and vibration means 3, which act on the same body cavity 4, such as a vagina, rectum, mouth, armpits, etc.

The previous generation means 1 and application means 2 are mutually enabled for transmission of the generated electromagnetic radiation from the generation means 1 to the application means 2, by physical means such as a cable, as schematically shown in figure 1, or also by wireless means.

Likewise, said application means 2 are enabled for communication and irradiation of the aforementioned electromagnetic radiation in the anatomical cavity 4 to be treated.

On the other hand, the vibration means or vibrator 3 are enabled for generation of a vibration. The vibrator 3 is also enabled to transmit by itself the generated vibration directly to the anatomical cavity to be treated, doing so separately from the application means 2, as schematically shown in figure 1.

According to the previous descriptions, the therapeutic intracavitary device of the proposed invention can have multiple preferred embodiments, responding to different user and patient needs.

Further explanations of different modalities and preferred embodiments of the same therapeutic intracavitary device of the invention will be provided below.

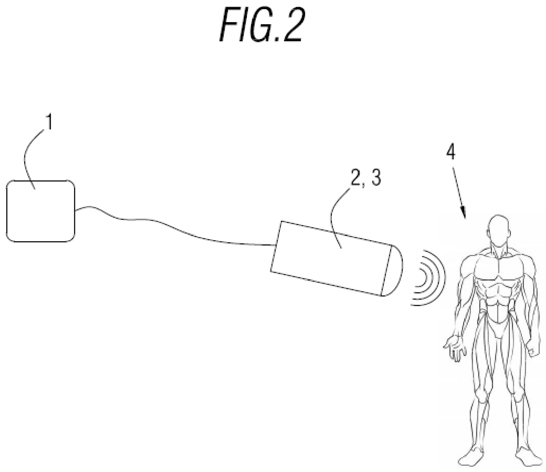

In other preferred embodiments of the same invention, the vibrator 3 may also be simultaneously and physically linked with the application means 2, so that the application means 2 and the vibrator 3 respectively communicate the radio frequency electromagnetic radiation and the vibration simultaneously from the same position to the same treated anatomical cavity 4. For this purpose, for example, the application means 2 and the vibrator 3 could be mutually integrated, as schematically shown in figure 2.

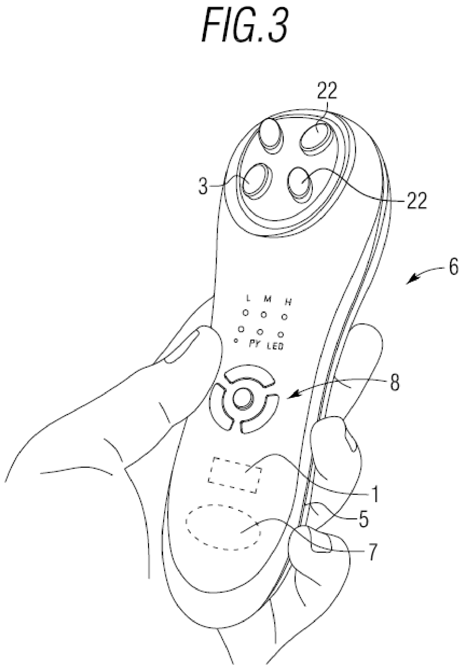

As schematically shown in figure 3, the therapeutic intracavitary device of the proposed invention may comprise manual gripping means by way of a knob 5 or handle.

On the other hand, the application means 2 for applying electromagnetic radiation may be applied to the skin and comprise at least one electrode 22 that could be independently controlled, and thus result in separate and distinct functions.

The knob 5 for manual gripping, the electrode 22 and the vibrator 3 may be arranged and integrated in a single body 6, thus allowing comfortable simultaneous manual operation by the user or specialised technician, as schematically shown in figure 3.

The aforementioned knob 5 or handle of the therapeutic intracavitary device of the invention may have a specially designed shape which advantageously allows the user or specialised technician to know the position of the same therapeutic intracavitary device of the invention at all times. This is because in the use of the therapeutic intracavitary device of the invention, when its active part is inserted into the corresponding body cavity 4, said active part is not visible and the user needs to know the position of its end part.

In different preferred embodiments, as also schematically shown in figure 3, the same body 6 may also include or integrate the generation means 1, and may also incorporate a rechargeable battery power supply (not shown in the drawings), which is enabled to properly supply power to the same generation means 1, the electrodes 22 and the vibrator 3, so as to be able to operate in alternative mode without the need to be permanently connected to a power supply grid.

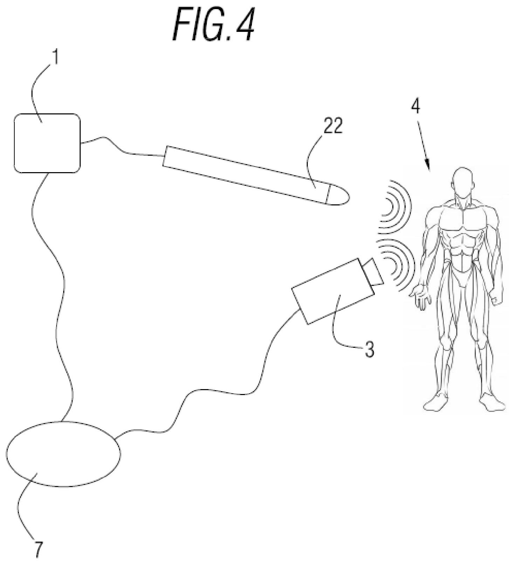

In the therapeutic intracavitary device of the invention, the generation means 1, the electrode 22 and the vibrator 3 may also be linked in data communication with control means 7 that can also control them, as schematically shown in figure 4.

The control means 7 may also be arranged in the same body 6 with interface means 8 for user control, as also shown in figure 3.

The electrode 22, which is linked with the generation means 1, applies electromagnetic radiation by means of radio frequency currents on the affected parts of the patient's body cavity 4.

Similarly, the vibrator 3 generates and applies a vibration on the affected parts of the patient's same body cavity 4.

By manually operating and gripping the body 6 by means of the knob 5, the user can then direct and apply a beam of radio frequency electromagnetic energy as well as a vibration to the area of application of the patient's cavity 4, which are absorbed by the tissues, leading to very beneficial therapy for various medical and aesthetic treatments.

The radio frequency may be in preferred frequency mode between 400 KHz and 3 MHz. The power and frequency supplied by the electrode 22 is controlled and regulated from the generation means 1 as controlled from the control means 7. Advantageously, the treatment can thus be customised to the needs of each patient.

Likewise, the power and frequency of the vibration generated by the vibrator 3 is controlled and regulated from the control means 7. Advantageously, the treatment can also thus be customised to the needs of each patient.

The control means or module 7 may be programmable according to the intensity and frequency range of the radio frequency current to be applied by the electrode 22, and also according to the intensity and frequency of the vibration of the vibrator 3, and the duration time of application of each of them in the treatment. In this way, the therapeutic intracavitary device of the invention could even be operated in automatic mode for a preset treatment time for a patient and suitably arranged and positioned relative to the body cavity 4 to be treated, without direct and/or permanent intervention by the user or specialised technician.

The electrodes 22 used in radio frequency transmission may be applied to the skin, and may be insulated or non-insulated, active and/or passive, and may be monopolar or bipolar return electrodes.

In monopolar electrodes, the radio frequency conducted is applied by means of two electrodes: one return electrode in a fixed position, incorporated in a passive plate that is located under the area being treated, and another active plate that is located on the other side of the area being treated and which can be in a fixed position, incorporated in an active or mobile plate, incorporated in an element that moves.

In bipolar electrodes, they include the active and passive part of the radio-frequency circuit in a single element. In turn, this bipolar electrode can be in a fixed position (bipolar plate) or a mobile position.

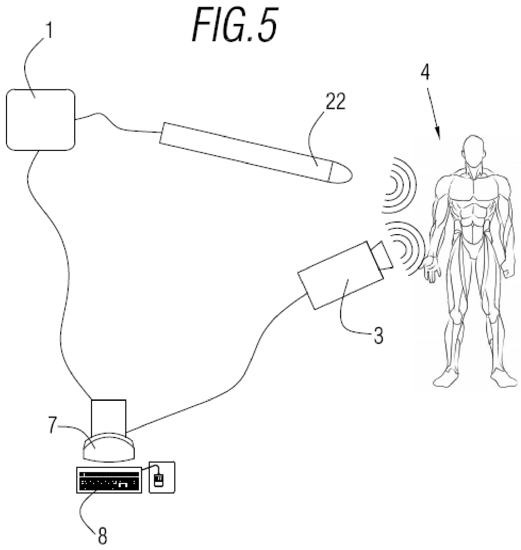

As schematically shown in figure 5, in other embodiments of the same invention, the control means 7 may be physically separated from the body 6 and comprise a laptop computer or similar, and the interface means 8 may comprise a touch display and/or keyboards of the same laptop computer or similar or other push buttons, which allows the frequency and intensity of the radio frequency electromagnetic radiation and vibration to be quickly displayed.

The computerised control module 7 is connected to the user interface 8 that integrates the display and the keyboard, connected to the generation means 1 with electrodes 22 and connected to the vibrator 3, thus allowing, after the necessary parameters have been previously entered, different treatments to be automated by following a complex pattern of times and temperatures that optimise the results, allowing the evolution of the treatment to be managed and, furthermore, being able to carry out all its phases without the need for operator intervention.

Said interface means 8 may be connected to the computerised control module 7 by a cable or by a wireless connection, such as Bluetooth or Wi-Fi.

The communication of the control means 7 with the generation means 1 and electrode 22 and with the vibrator 3 could be carried out by a cable, or even wirelessly. Remote control of the control means 7 from a mobile phone or similar could also be enabled by means of an application for this purpose.

To facilitate the different treatments, the control module 7 could comprise a removable memory card on which the details of the particular treatments of the various patients, the number of sessions or the type of sessions to be applied can be stored. Furthermore, said exchangeable memory card can act as a safety element, so that the therapeutic intracavitary device of the invention can only function when a memory card is inserted.

On the other hand, the control means 7 may incorporate a specific software program for this purpose, which allows synchronised operation of the electrodes 22 and the vibrator 3 of the therapeutic intracavitary device of the invention, assigning a suitable power value and frequency.

The computerised control module 7, by means of its software, can automatically regulate and/or stop the flow and intensity of the radio frequency and vibration to be transferred, depending on different particular parameters of the patient to be treated.

Even in other preferred embodiments of the same invention, the generation means 1, the electrode 22 and the vibrator 3 could be arranged and linked together without the precise condition of being manually operable, but operable by means of automated means, for automatic and remote and/or robotic operation and service controlled from the control means 7, as can also be deduced from the schematic representations of figures 1, 2, 4 and 5.

This could also be compatible so that they are also integrated in a body 6, for example, but this body is designed to act on the patient without the need to be manually operated by a user or specialised technician, but automatically and/or remotely and/or robotically by means of the control means 7, for example.

The therapeutic intracavitary device of the invention may also incorporate a temperature sensor (not shown in the drawings). Said temperature sensor could be linked in data communication with the control means 7 in order to optimise the intensity and frequencies of the radio frequency and vibration in accordance with the measured temperature.

In accordance with the above, the computerised control means 7 and its computer program or installed software, connected to the patient interface 8, which may integrate a touch display, as well as to the generation means 1 and electrode 22 and the vibrator 3, thus allow, after operating parameters suitable for a specific treatment have been previously entered, the different treatments to be automated by following a determined pattern of times and temperatures that optimise the results, so as to manage the evolution of the treatment and even, according to different preferred embodiments of the same invention, be able to carry out all its phases even without the need for direct or permanent intervention by any user or specialised technician.

The temperature sensors may also be linked to the electrodes 22 and/or to the vibrator 3, and thus regulate their operation in intensity and frequency.

In other preferred embodiments of the same invention, the generation means 1 and the control means 7 may be mutually integrated.

The invention further includes a method of therapeutic intracavitary treatment, designed for treatment and therapy in a human or animal anatomical cavity. This method, also included in the invention and in different preferred embodiments, could also be carried out with a therapeutic intracavitary device of the same invention.

The characteristic feature of said method is that it involves irradiation of electromagnetic radiation in the anatomical cavity and at the same time applying vibration on the same anatomical cavity.

Due to their particular equipment, the therapeutic intracavitary device of the invention, as well as the method of therapeutic intracavitary treatment included in the same invention, allow a therapeutic use and treatment based on current or electromagnetic fields of different frequency, and at the same time a therapeutic use and treatment based on an application of vibrations, in such a way that both techniques and types of treatment are made compatible while maintaining all their properties and advantages on the anatomical cavity 4 to be treated.

The therapeutic intracavitary device of the invention, as well as the method of therapeutic intracavitary treatment included in the same invention, allow a design and structural configuration that are specifically devised for an ergonomic intracavitary use in order to apply physiotherapeutic treatments by means of molecular stimulation, serving as an instrument in the treatment or relief of very different conditions.

Due to their particular design, the therapeutic intracavitary device, as well as the method of therapeutic intracavitary treatment included in the same invention, can be adapted in their configuration and operation, according to the different anatomical cavities 4 to which the application of their treatment is intended, in order to allow for said application in the best possible way.

In accordance with all the above descriptions, the therapeutic intracavitary device of the invention, as well as the method of therapeutic intracavitary treatment included in the same invention, have the advantage of being able to simultaneously provide the treated anatomical cavity 4 with both radio frequency electrotherapy treatment and treatment on the same anatomical cavity 4 treated by means of vibration, with all the advantages and benefits that this entails.

The details, shapes, dimensions and other accessory elements, as well as the materials used in the manufacture of the therapeutic intracavitary device may be suitably substituted with others that are technically equivalent and do not depart from the essential features of the invention or from the scope defined by the claims included below.

## Claims

1. A therapeutic intracavitary device, enabled for use in a human or animal anatomical cavity (4); **characterised in that** it comprises generation means (1) for generating electromagnetic radiation, application means (2) for applying said electromagnetic radiation, and vibration means (3); the generation means (1) and the application means (2) being mutually enabled for transmission of the electromagnetic radiation to the application means (2); said application means (2) being enabled for communication and irradiation of the electromagnetic radiation in the anatomical cavity (4); wherein the vibration means or vibrator (3) are enabled for generation of a vibration and for communication of the vibration in the same anatomical cavity (4).

2. The therapeutic intracavitary device according to claim 1, wherein the application means (2) comprise at least one electrode element (22).

3. The therapeutic intracavitary device according to any of the preceding claims, wherein the generation means (1), the application means (2) and the vibrator (3) are linked in data communication with control means (7) that can control them.

4. The therapeutic intracavitary device according to any of the preceding claims, comprising manual gripping means, said gripping means and the application means (2) and/or the vibrator (3) being mutually enabled for manual operation.

5. The therapeutic intracavitary device according to claim 3 or 4, wherein the control means (7) are programmable and incorporate a program or software enabled for control over the generation means (1), the application means (2) and the vibrator (3).

6. The therapeutic intracavitary device according to any of claims 3 to 5, when they are dependent on claim 2, wherein the electrodes (22) may be applied to the skin and/or may be insulated or non-insulated.

7. The therapeutic intracavitary device according to any of claims 3 to 6, when they are dependent on claim 2, wherein the electrodes (22) may be of the active and/or passive type.

8. The therapeutic intracavitary device according to any of claims 3 to 7, when they are dependent on claim 2, wherein the electrodes (22) are monopolar or bipolar return electrodes.

9. The therapeutic intracavitary device according to any of the preceding claims, incorporating a temperature sensor.

10. The therapeutic intracavitary device according to claim 9, wherein the temperature sensor is linked in data communication with the control means (7), and/or with the application means (2), and/or with the vibrator (3).

11. The therapeutic intracavitary device according to any of the preceding claims, wherein the electromagnetic radiation is by radio frequency.

12. The therapeutic intracavitary device according to any of the preceding claims, wherein the electromagnetic radiation applied from the application means (2) is at a frequency between 400 KHz and 3 MHz.

13. A method of therapeutic intracavitary treatment, designed for therapeutic treatment of a human or animal anatomical cavity, **characterised in that** it comprises irradiation of electromagnetic radiation in the anatomical cavity and at the same time applying vibration on the same anatomical cavity.

14. The method of therapeutic intracavitary treatment according to claim 13, wherein the electromagnetic radiation is by radio frequency.

15. The method of therapeutic intracavitary treatment according to claim 13 or 14, wherein the irradiated electromagnetic radiation is at a frequency between 400 KHz and 3 MHz.
